(19) Europäisches Patentamt
European Patent Office
Office européen des brevets

(11) Publication number : **0 378 246 B1**

(12) # EUROPEAN PATENT SPECIFICATION

(45) Date of publication of patent specification : **11.03.92 Bulletin 92/11**

(51) Int. Cl.$^5$ : **A61K 31/785**

(21) Application number : **90102530.4**

(22) Date of filing : **18.06.86**

(54) **Biologically active copolymers.**

(30) Priority : **18.06.85 US 745917**
**15.10.85 US 787770**
**13.06.86 US 872111**

(43) Date of publication of application :
**18.07.90 Bulletin 90/29**

(45) Publication of the grant of the patent :
**11.03.92 Bulletin 92/11**

(84) Designated Contracting States :
**AT BE CH DE FR GB IT LI LU NL SE**

(56) References cited :
**US-A- 4 323 467**
**FEDERAL PROCEEDINGS, vol. 44, March 1985, page 1710, article 7596; T.P. ATKINSON: "Induction of suppressor cells by reverse block copolymers"**

(56) References cited :
**THE JOURNAL OF IMMUNOLOGY, vol. 133, no. 6, December 1984, pages 3167-3175, Williams and Wilkins, Baltimore, US; R.L. HUNTER et al.: "The adjuvant activity of nonionic block polymer surfactants"**

(60) Publication number of the earlier application in accordance with Art. 76 EPC : **0 228 448**

(73) Proprietor : **EMORY UNIVERSITY**
**1380 South Oxford Road**
**Atlanta, GA 30322 (US)**

(72) Inventor : **Hunter, Robert L.**
**3640 Churchwell Court**
**Tucker, Ga. 30084 (US)**

(74) Representative : **Sternagel, Hans-Günther, Dr. et al**
**Patentanwälte Dr. Michael Hann Dr. H.-G. Sternagel Sander Aue 30**
**W-5060 Bergisch Gladbach 2 (DE)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to a further medical use of specific octablock copolymers and more particularly to the use of those compounds for the preparation of pharmaceutical compositions.

R. Hunter et al. describe in the Journal of Immunology, Vol. 133, No. 6. December 1984, pages 3167 to 3175 the adjuvant activity of nonionic octablock copolymer surfactants. The surfactants are block copolymers of hydrophilic polyoxyethylene and hydrophic polyoxypropylene units and having four active hydroxyl groups. The compounds produce a distinct pattern of immune response and inflammation when used in pharmaceutical compositions.

T.P. Atkinson et al. reported in Federal Proceedings, Vol. 44, March 1975, Article 7596, page 1710 on the induction of suppressor cells by the block copolymers described in Journal of Immunology, Vol 133. The suppression of antibody formation to immunogenic preparations of bovine serum albumin (BSA) is mentioned.

### Immune Stimulating Compounds

The immune system is a highly complex system of cells and tissues that requires the cooperation of a large number of different cell types. The systems of the body that make up the immune system network are variously categorized as belonging to the hematopoietic system, the reticuloendothelial or the phagocytic system and the lymphoid system.

The hematopoietic system is located in the bone marrow and is responsible for supplying the various precursor and accessory cells of the immune systems. The reticuloendothelial system is made up of the phagocytic cells that are responsible for destroying or neutralizing foreign material that may enter the body. The lymphoid system is made up of lymphocytes, and is responsible for the overall regulation of the immune system and for the production of antibodies.

The tissues of the lymphoid system are generally classified as the central tissues and the peripheral tissues. Two central lymphoid tissues of mammals are bone marrow and thymus. In addition, fowl have a third central lymphoid organ, the bursa of Fabricius, which is critical to the development of the immunoglobulin-producing cells. It is thought that the mammals have a bursal equivalent associated with the intestinal tract. Lymph nodes, spleen, tonsils, intestinal lymphoid tissue (Peyer's patches) and other collections of lymphocytes constitute the peripheral lymphoid tissues.

In mammals, the bone marrow, if considered as a single tissue, is the largest tissue of the body. In the average human adult the total weight of the bone marrow is about 3 kg. Marrow fills the central core of nearly all bones. Bone marrow has three types of tissue; vascular tissue, adipose tissue and the tissue directed to hematopoiesis or blood cell formation. The vascular tissue is the circulatory system that supplies nutrients and removes wastes from the actively growing cells. The hematopoietic tissue is responsible for the formation of erythrocytes, platelets, granulocytes and monocytes, and lymphocyte precursors. Adipose tissue consists of fat cells which contribute little to the function of the bone marrow.

The other central lymphoid tissue is the thymus; a bilobed organ situated in the anterior thoracic cage over the heart. In other species, the thymus may be distributed along the neck and thorax in several lobules.

Embryologically, the thymus emerges from the third and fourth branchial pouches. The human thymus is a fully developed organ at birth and weighs 15 to 20 grams. By puberty it weighs 40 grams, after which it atrophies or involutes becoming less significant structurally and functionally. Atrophy of the thymus with age is a characteristic of all species which is associated with aging and the cessation of growth. The incidence of age related diseases increases as the thymus shrinks and thymus-dependent immunity decreases. This age-associated decrease in thymic weight, called involution, is accompanied by changes in the thymic structure and a general decline in thymic function. Transient involution of the thymus may also occur as a consequence of a stress or infection. Thymic involution may be controlled hormonally; castration slows involution while injection of corticosteroid hormones accelerates involution. Numerous studies have demonstrated that the thymic involution associated with increasing age parallels a reduction of T-lymphocyte-mediated immunity and increased incidence of diseases associated with aging. Many diseases and treatments can accelerate involution of the thymus; virtually none are known to enhance growth of the thymus or reverse involution.

Anatomically, the thymus is a pouch of epithelial cells filled with lymphocytes, nourished and drained by the vascular and lymphatic systems and innervated by the autonomic nerves. The epithelial cells and other structural cells divide the thymus into a complex assembly of continuous lobes, each of which is heavily laden with lymphocytes. The epithelial cells produce hormones and regulate some of the activities of the lymphocytes. The lymphocyte population is greatest in the cortex or outer portion of each lobule. The inner section, the medulla, has more epithelial cells and fewer lymphocytes but the lymphocytes are more mature.

Lymphocytes can generally be classified as either T-lymphocytes or as B-lymphocytes. B-lymphocytes are

responsible for the production of antibodies (immunoglobulin) in response to a challenge by a particular antigen. T-lymphocytes are responsible for the general regulation of the immune system and are also the principal mediators in cell-mediated immune responses. They also influence the proliferation of bone marrow cells and are probably involved in the growth and differentiation of other organs as well.

All lymphocytes are ultimately derived from stem cells in bone marrow. These lymphocyte precursors are dispersed into the blood where they course through many organs. However, critical events take place in the thymus and bursa of Fabricius (or its mammalian equivalent) that imprint the lymphocytes with special functions and that regulate the development into either T or B-lymphocytes.

Life-Span studies of lymphocytes of most mammalian species divide lymphocytes into two fractions - those with a short span (mostly large lymphocytes) of 5 to 7 days and the small lymphocytes with a life span measured in months or even years. The former are usually B-lymphocytes and the latter are usually T-lymphocytes.

B-lymphocytes respond to immunologic phenomena very differently from a T-lymphocyte in practically every instance. T-lymphocytes are formed in the thymus from lymphoblasts that left the bone marrow. This maturation is expressed morphologically as a reduction in cell size to about 7 μm in diameter. The thymic cortex is rich in lymphocytes of all sizes. These thymocytes are not morphologically distinguishable from lymphocytes in other tissues, but they are immature and antigenically identifiable by the presence of several cell surface antigens including the ∅, or T antigen, a distinctive surface marker antigen that separates the T-lymphocyte from the B-lymphocyte.

Enumeration of lymphocytes indicate that 65% to 85% of all lymphocytes in the blood are of the T type. Lymphocytes of the thoracic duct fluid are nearly 90% to 95% of the T variety and those in the Peyer's patches or the gut are 50% to 65% T-lymphocytes. The T-lymphocyte population of lymph nodes, particularly in the deep cortical region, is high, but is low in the tonsil and the appendix.

When the T-lymphocyte contacts a recognizable antigen in the appropriate context, it passes through a phase of growth and cell division known as lymphocyte transformation to produce a large population of its own kind. The antigen must first be "processed" by macrophages and then presented to T-lymphocytes.

T-lymphocytes are actually divided into several subsets and the role that they play in the immune system is complex. The T-lymphocyte is responsible for the phenomenon known as the cell-mediated immune response. In a cell-mediated immune response, the T-lymphocytes that recognize a cell-bound antigen begin producing and secreting a wide variety of proteins that affect the activity of other types of cells in the immune system. These proteins include lymphokines that attract, activate and hold phagocytes at the site of the antigen and interferons that provide protection against virus infection.

The T-lymphocyte is also an important regulator of B lymphocyte function. The antigen-exposed T-lymphocyte may have either of two direct and opposite effects on B-lymphocytes depending on the subclass of T-lymphocyte. The major subclasses are the helper cell and the other is the suppressor cell. Helper T-lymphocytes are necessary for a complete B cell response to T-lymphocyte-dependent antigens. T-lymphocyte dependent antigens tend to be the more complex antigens such as bacterial proteins, virus proteins and other large complex proteins in general.

Unlike helper T-lymphocytes, suppressor T-lymphocytes block the development of effector B and T-lymphocytes. Specific suppressor T-lymphocytes have now been demonstrated to play a large role in tolerance to many proteins, both in antibody and cell-mediated immune responses. In addition, genetic unresponsiveness to some antigens is due to the greater stimulation of suppressor T-lymphocytes than of helper T-lymphocytes by these antigens.

Thus, in the normal, healthy animal, the thymus is normally active only during the early years of life. During these early years of thymic activity, the thymus supplies the animal with the T-lymphocytes which will serve the animal for the rest of its life. In certain diseases, such as rheumatoid arthritis, the thymus may regain some activity during adult life. This demonstrates that the adult thymus retains capacity to function and that involution is not necessarily permanent. At least partial function might be restored if the appropriate agents were available.

Acquired T-lymphocyte deficiency diseases of adults are characterized by a depletion of circulating T-lymphocytes. The symptoms expressed in these diseases include an inability to mount a cell mediated immune response in response to an antigen challenge. An example of an acquired T-lymphocyte deficiency disease is acquired immune deficiency syndrome or AIDS.

AIDS is a disease caused by the human T-lymphocyte lymphotropic virus (LAV or HTLV-III). The virus specifically attacks T-4 helper lymphocytes, a subgroup of T-lymphocytes that plays a major role in defending the body against infectious diseases. Depletion of this subset of lymphocytes is manifested by an increased incidence of opportunistic infections like *pneumocystis carinii* and certain cancers. More specifically, the virus enters the T-lymphocyte and incorporates viral encoded DNA into the DNA of the host T-lymphocyte. As long as the infected T-lymphocyte remains inactivated, the virus will quietly remain in the DNA of the host cell. This will not kill the cell but may impair its function. When the infected T-lymphocytes are activated by stimuli such

as a specific antigen, the viral DNA in the host DNA is expressed and produces new viral particles. The host T-lymphocyte is then killed and lysed, releasing new viral particles that can invade and kill other T-lymphocytes. The loss of T-4 lymphocytes is profound and occurs even faster than can be accounted for by direct viral killing of the cells. This has led some investigators to postulate that the infection somehow shuts off the production of T-4 lymphocytes. In any case, the thymus in the normal adult is no longer functioning and the killed T-lymphocytes cannot be replaced leaving the patient vulnerable to subsequent infections. Especially striking are recent studies of the thymuses of deceased AIDS patients ranging in age from 10 months to 42 years. AIDS victims have profound thymic involution; much more extensive than in age-matched patients who died of other causes.

The cure of a person with AIDS will probably require one agent to eliminate the virus and other agents to cause the body to replace T cells that have been killed by the virus. The first step is to eliminate the AIDS virus from the patient. This will have to be supported by other therapies to induce restoration of immune function. Studies to date with macrophage activating agents, interferon inducers and lymphokines have been disappointing, possibly because their targets, T-lymphocytes, do not exist in sufficient numbers. Interleukin 2 restores the function of one subset of non T-cells (natural killer cells) but has no effect on a host of other serious defects. More drastic measures can be performed. One potential method of restoring the immune system is by transplanting bone marrow from healthy donors. However, this is a dangerous procedure. It may produce lethal graft versus host disease unless the patient's donor is an identical twin.

Another area where there is a need to re-establish not only the immune system, but also the hematopoietic system, is in total body irradiation for treatment of leukemia. When a patient undergoes high dose total body irradiation, the entire immune system is destroyed. The usual treatment after the irradiation is to perform a bone marrow transplant with marrow from a close relative. If the transplant is successful, the new marrow will produce new cells, thereby restoring both red blood cells and whited blood cells to the body. However, this is a dangerous treatment that is successful in only a fraction of the cases. Localized radiation of tumors and several types of chemotherapy also produce suppression T-cell mediated immunity.

What is needed is a safe and effective method of re-establishing T-lymphocyte function. One method of re-establishing T-lymphocyte function is by treating existing T-lymphocytes so that they resume their normal immune functions. Agents that have been shown to be effective in certain situations in stimulating T-lymphocytes include macrophage activating factors, interferon inducing agents, lymphokines and cytokines. However, in a disease such as AIDS or in the case of irradiation in which the T-lymphocyte population has been destroyed, this type of treatment is not effective because the number of T-lymphocytes is severely depleted. In these cases, an effective method of causing the thymus to produce new T-lymphocytes would be the treatment of choice. However, to date, there is no effective treatment that will cause the thymus to reverse the process of involution and produce new T-lymphocytes.

**Autoimmune diseases**

Autoimmune diseases are characterized by the development of an immune reaction to self components. Normally, tissues of the body are protected from attack by the immune system; in autoimmune diseases there is a breakdown of the self-protection mechanisms and an immune response directed to various components of the body ensues. Autoimmune diseases are for the most part chronic and require life long therapy. The number of recognized autoimmune diseases is large and consists of a continuum ranging from diseases affecting a single organ system to those affecting several organ systems. With increased understanding of the molecular basis of disease processes, many more diseases will likely be found to have an autoimmune component. Specific examples of autoimmune diseases are presented below.

**Spectrum of Autoimmune Diseases**

| | |
|---|---|
| Organ Specific | Hashimoto's thyroiditis |
| | Graves' disease |
| | Addison's disease |
| | Juvenile diabetes (Type I) |
| | Myasthenia gravis |
| | Pemphigus vulgaris |
| | Sympathetic opthalmia |
| | Multiple sclerosis |
| | Autoimmunehemolytic anemia |
| | Active chronic hepatitis |

| | |
|---|---|
| | Rheumatoid arthritis |
| Non-organ specific | Systemic lupus erythematosus |

Systemic lupus erythematosus (SLE) is an inflammatory, multisystem disease characterized clinically as a relapsing disease of acute or insidious onset that may involve any organ in the body. Clinically, symptoms are due to disease affecting the skin, kidneys, serosal membranes, joints and heart. Anatomically, all sites have in common vascular lesions with fibrinoid deposits and immunologically, the disease involves antibodies of autoimmune orgin, especially antinuclear antibodies (ANA). The ANA are directed against both DNA and RNA. Autoantibody development appears to be multifactorial in orgin, involving genetic, hormonal, immunologic and environmental factors.

The morphologic changes seen in organs result from the formation of circulating immune complexes and their deposition in a variety of tissues. Although many organs can be affected, some are affected more than others. Lesion of joints, the kidneys, heart, and serous membranes are responsible for most of the clinical signs. The course of SLE is extremely variable and unpredictable. An acute onset with progressive downhill course to death within months can occur. The usual course however, is characterized by flareups and remissions spanning a period of years or even decades. It usually arises in the second or third decades of life, but may become manifest at any age.

Acute attacks are usually treated by adrenocortical steroids or immunosuppressive drugs. These drugs often control the acute manifestations. With cessation of therapy the disease usually reexacerbates. The prognosis has improved in the recent past; approximately 70 to 80% of patients are alive 5 years after the onset of illness and 60% at 10 years. Lifelong therapy is required to control the disease.

At one time SLE was considered to be a fairly rare disease. Better methods of diagnosis and increased awareness that it may be mild and insidious have made it evident that its prevalence may be as high as 1 case per 10,000 population. There is a strong female preponderance - about 10 to 1.

Rheumatoid arthritis is a systemic, chronic, inflammatory disease that affects principally the joints and sometimes many other organs and tissues throughout the body. The disease is characterized by a nonsuppurative proliferative synovitis, which in time leads to the destruction of articular cartilage and progressive disabling arthritis. The disease is caused by persistent and self-perpetuating inflammation resulting from immunologic processes taking place in the joints. As is the case with most autoimmune diseases, the trigger that initiates the immune reaction remains unidentified. Both humoral and cell mediated immune responses are involved in the pathogenesis of rheumatoid arthritis. The majority of patients have elevated levels of serum immunoglobulins and essentially all patients have an antibody called rheumatoid factor (RF) directed against a component of another antibody class.

The key event in the pathogenesis of the arthritis is the formation of antibodies directed against other self antibodies. Why these antibodies are formed is unknown at present. It has been suggested that the process is initiated by the formation of antibodies or immunoglobulins against an unknown antigen, possibly an infectious agent. When the antibodies combine with the antigen, conformational changes occur in a portion of the antibody molecule creating new antigenic determinants. The appearance of new determinants evokes an antibody respond against the antibody molecule and results in the formation of anti-immunoglobulin antibodies or rheumatoid factor. T cells may also be involved in the pathogenesis of rheumatoid arthritis. A large number of T cells are found in the synovial membrane, outnumbering B cells and plasma cells. Additionally, procedure to decrease the population of T cells (such as draining the thoracic duct), result in remission of symptoms.

The most destructive effects of rheumatoid arthritis are seen in the joints. Classically, it produces symmetric arthritis, which principally affects the small joints of the hands and feet, ankles, knees, wrists, elbows, shoulders, temporo-mandibular joints and sometimes the joints of the vertebral column. The clinical course is highly variable. After approximately 10 years, the disease in about 50% of the patients becomes stabilized or may even regress. Most of the remainder pursue a chronic, remitting, relapsing course. After 10 to 15 years, approximately 10% of patients become permanently and severely crippled. The disease usually has its onset in young adults but may begin at any age and is 3 to 5 times more common in women than in men.

Rheumatoid arthritis is a very common disease and is variously reported (depending on diagnostic criteria) to affect 0.5 to 3.8% of women and 0.1 to 1.3% of men in the United States.

Multiple sclerosis is another disease that is thought to be caused by autoimmune mechanisms. The cause of multiple sclerosis is unknown but seems to be multifactorial. Susceptibility or resistance may be genetically determined; something in the environment interacts with the human host at the proper age to cause biochemical and structural lesions in the central nervous system. The systemic immune response and the response of the central nervous system become involved. Although the cause and pathogenesis of multiple sclerosis are unknown, it is widely believed that immune abnormalities are somehow related to the disease. Three possible mechanisms have been postulated: infection, autoimmunity, and a combination of the two. Suppression or modulation of the immune responses may be the key.

The graphic distribution of multiple sclerosis indicate that the disease is acquired from an environmental factor. Approximately 200 studies of the geographic distribution of multiple sclerosis have been conducted and have shown that regions of high prevalence (30 to 80 cases per 100,00 population) in northern Europe between 65 and 45 degrees north latitude and in the northern United States and southern Canada as well as in southern Australia and New Zealand. In contrast, regions of low risk, including most of Asia and Africa, have a prevalence of 5 or fewer cases per 100,000.

Myasthenia gravis is an autoimmune disorder caused by antibodies directed against the acetylcholine receptor of skeletal muscle. Present information indicates at least three mechanisms whereby acetylcholine receptor antibody may interfere with neuromuscular transmission and thus induce myasthenia gravis. Acetylcholine receptor antibody may interfere (directly or indirectly) with acetylcholine receptor function. In both experimental allergic myasthenia gravis and human myasthenia gravis, the extent of acetylcholine receptor loss parallels the clinical severity of the disease, suggesting that acetylcholine receptor antibody-induced acceleration of acetylcholine receptor degradation is important in the development of myasthenia gravis. Complement-mediated destruction of the postsynaptic region is the third possible cause. Other disorders, especially those presumed to be autoimmune in origin, can occur in association with myasthenia gravis. Thyroid disease, rheumatoid arthritis, systemic lupus erythematosus, and pernicious anemia all occur more commonly with myasthenia gravis than would be expected by chance. The prevalence of myasthenia gravis in the United States is one per 20,000.

The foundation of therapy of autoimmune diseases is treatment with immunosuppressive agents. The basis for this therapy is attenuation of the self-directed immune response with the primary aim being to control symptoms of the particular disease. The drugs utilized to achieve this aim are far from satisfactory, in that adverse side effects are numerous and control of the disease is many times difficult to achieve. The problem is compounded by the chronicity of the disease with effective therapy becoming more difficult with time. An indication of the severity of particular diseases is seen in the willingness to accept greater risks associated with therapy as the disease progresses. Currently available therapy is distinctly non-selective in nature, having broad effects on both the humoral and cell mediated arms of the immune system. This lack of specificity can limit the effectiveness of certain therapeutic regimens. The main groups of chemical immunosuppressives are alkylating agents, antimetabolites, corticosteroids, and antibiotics, each will be discussed briefly.

The corticosteroids, also called adrenocorticosteroids, are fat-like compounds produced by the outer layer or cortex, of the adrenal gland. The adrenal cortex is an organ of homeostasis influencing the function of most systems in the body. It is responsible for adaptation of the body to a changing environment. Therapeutic use of the corticosteroids for autoimmune disease is based on their two primary effects on the immune system, anti-inflammatory action and destruction of susceptible lymphocytes. They also effect a redistribution of lymphocytes from peripheral blood back to the bone marrow. The use of corticosteroids is not without adverse side effects however, particularly during the course of life-long treatment which is required for many of the autoimmune diseases. Major side effects of steroids are:

1. Cushing syndrome
2. Muscle atrophy
3. Osteoporosis
4. Steroid induced diabetes
5. Atrophy of the adrenal glands
7. Interference with growth
7. Susceptibility to infections
8. Aseptic bone necrosis
9. Cataract development
10. Gastric ulcer
11. Steroid psychosis
12. Skin alterations
13. Nervous state accompanied by insomnia

Attempts to minimize side effects incorporate alternate day or less frequent dosage regimens.

A recently developed immunosuppressive agent is the antibiotic cyclosporin A. The antibiotic has greatest activity against T cells and does not seem to have much direct effect on B cells. The drug is being evaluated for the treatment of autoimmune diseases for which it shows some promise. Side effects include hair growth, mild water retention, renal toxicity, and, in older patients, nervous system disorders symptoms have been observed.

Other drugs are used alone or in combination with those listed above and include gold salts and antimalarials, such as chloroquine. Another class of drugs, the non-steroidal anti-inflammatory drugs are used extensively in arthritis. These drugs provide analgesia at low doses and are anti-inflammatory after repeated

administration of high doses. Nonsteriodal anti-inflammatory drugs all act rapidly and their clinical effects decline promptly after cessation of therapy. They do not prevent the progression of rheumatoid arthritis and do not induce remissions. Immunostimulants, such as levamisol have also been used in many autoimmune diseases but side effects have generally limited their use.

The object of the invention is the use of biologically active octablock copolymers having the following formula

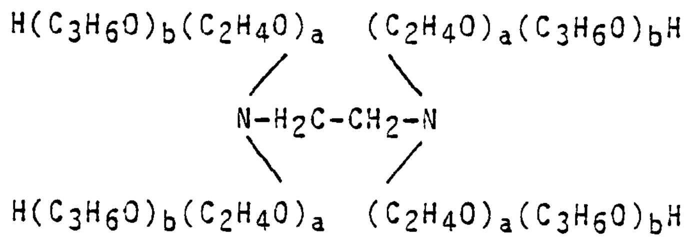

and

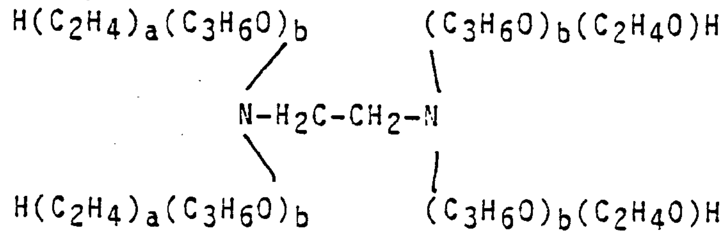

wherein:

the mean aggregrate molecular weight of the portion of the octablock copolymer represented by polyoxypropylene is between 5000 and 7000 daltons;

a is a number such that the portion represented by polyoxyethylene constitutes between 10 % to 40 % of the compound by weight; and

b is a number such that the polyoxypropylene portion of the total molecular weight of the octablock copolymer constitutes between 60 % and 90 % of the compound by weight for the preparation of a pharmaceutical composition for stimulating the immune system of an animal or human.

The biologically active copolymers have a wide variety of effects on individual cells. These compounds have ionophoric activity, *i.e*, they cause certain ions to be transported across cell membranes. The compounds can cause non-cytolytic mast cell degranulation with subsequent histamine release.

The pharmaceutical compositions comprising the biologically-active copolymers of the present invention can be administered orally to animals

The biologically-active copolymers can also be added to cattle feed to effect a change in the population of microorganisms normally resident in the rumen. Under normal conditions, the microorganisms digest the cellulose that is eaten by the cattle to the end-product methane. Methane is essentially unusable by the cattle. By administering the biologically-active copolymers of the present invention orally to the cattle, the copolymer differentially affects the rumen microorganism so that there is a shift in the rumen population of microorganisms resulting in an increase in proprionic acid production and a decrease in lactic acid and methane. Cattle are capable of using proprionate in their own metabolism thereby increasing the efficiency of food conversion.

Biologic effects of the copolymers vary with the structure of the polymer. The ability to modify the structure of these polymers to optimize particular biologic effects provides the potential to design synthetic compounds with a precision and ease not possible in other systems.

The biologically-active copolymer comprises a copolymer of polyoxyethylene (POE) which is hydrophilic and polyoxypropylene (POP) which is hydrophobic. The block copolymer is built on a tetrafunctional ethylenediamine initiator. In the preferred embodiment the biologically-active copolymers have the following general formulas:

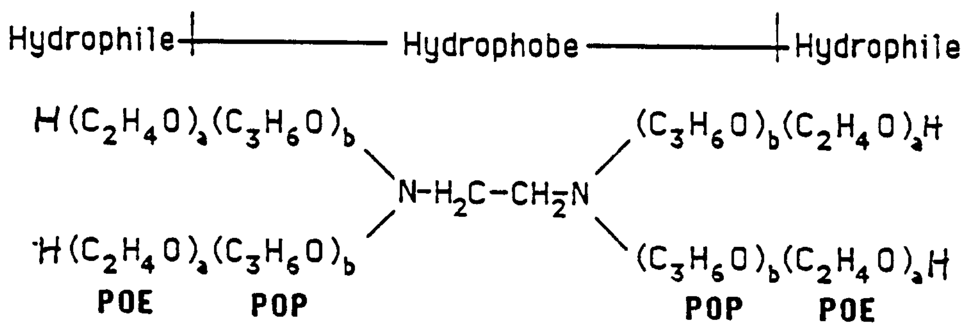

wherein:

the mean aggregate molecular weight of the hydrophobe portion of the octablock copolymer consisting of polyoxypropylene $(C_3H_6O)_b$ (POP) is between approximately 5000 and 7000 daltons;

a is a number such that the hydrophile portion represented by polyoxyethylene $(C_2H_4O)_a$ (POE) constitutes between approximately 10% and 40% of the total molecular weight of the compound; and

b is a number such that the polyoxypropylene $(C_3H_6O)_b$ (POP) portion of the total molecular weight of the octablock copolymer constitutes between approximately 60% and 90% of the compound.

In another embodiment of the biologically-active copolymer, the block copolymer comprises a polymer of hydrophilic polyoxyethylene (POE) built on an ethylene diamine initiator. Polymers of hydrophobic polyoxypropylene (POP) are then added to block of hydrophilic polyoxyethylene (POE). This results in an octablock copolymer with the following general formula:

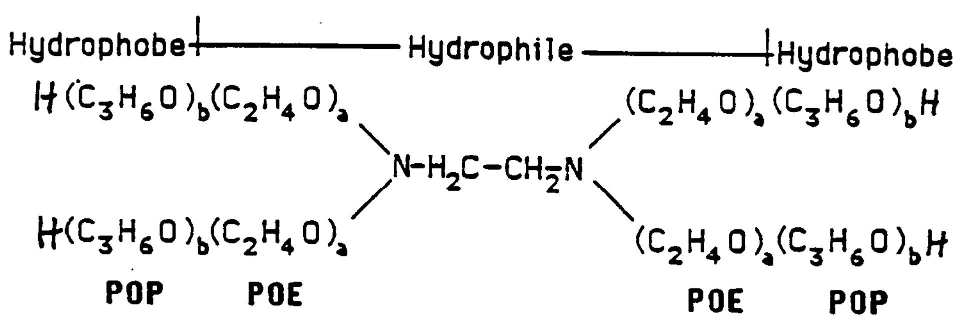

wherein:

a is a number such that the hydrophile portion represented by polyoxyethylene $(C_2H_4O)_a$ (POE) constitutes between 10% to 40% of the total molecular weight of the compound;

the mean aggregate molecular weight of the hydrophobe portion of the octablock copolymer consisting of polyoxypropylene $(C_3H_6O)_b$ (POP) is between 5000 and 7000 daltons; and

b is a number such that the polyoxypropylene $(C_3H_6O)_b$ (POP) portion of the total molecular weight of the octablock copolymer constitutes between 60% and 90% of the compound. The composition comprising the biologically-active copolymer is usually administered by a subcutaneous, intravenous, or intramuscular injection of an effective amount of the copolymer into an animal or human. The biologically-active copolymer can be taken orally if it is desired that the copolymer have an effect on alimentary canal microorganisms. Normally, very little of the copolymer is absorbed from the alimentary canal.

The compositions prepared according to the present invention stimulate the T-cell immune system.

These advantages of the present invention will become apparent after a review of the following detailed description of the disclosed embodiment and the appended claims.

The present invention uses a class of biologically active copolymers which have a wide variety of biological functions which are useful in treating various pathological conditions in both humans and animals and which can also be used to increase the efficiency of food production.

The biologically-active copolymers comprise a surface active compound with hydrophobic segments and a small proportion of hydrophile that is built upon an initiator compound. The initiator compound typically has one or more active hydrogens upon which the hydrophobic or hydrophilic polymer units are condensed. Compounds that can be used as initiators in producing the biologically active copolymers of the present invention include methylamine, ethylamine, propylamine, butylamine, amylamine, hexylamine, aniline, the alkylene polyamines, especially aliphatic primary diamines such as ethylenediamine, propylene diamine, diethylenetriamine, triethylenetetramine, tetraethylenepentamine, hexamethylene-diamine and phenylenediamine. Alkanolamines such as monoethanolamine, diethanolamine, triethanolamine, isopropanolamine, tri(t-propanol-)amine, 2-amino-1-butanol, N-butyl-di(2-propanol)amine can also be used as initiators. Furthermore, heterocyclic compounds containing a hetero nitrogen atom can be employed, such as piperazine, 2 methylpiperazine, 2,5-dimethylpiperazine, imidazimidazole, pyrazolidine, pyrazolidone, hydantoin and dimethyl hydantoin. Hydroxyl amine and the hydroxylamine derivatives and aminophenol and aminophenol derivatives can also be used as initiators to produce the biologically active copolymers used for the present invention.

The compositions of this invention comprise surface active mixtures of conjugated polyoxypropylene-polyoxyethylene compounds based on the reactive hydrogen compounds wherein chains of oxypropylene groups having a defined molecular weight are attached to the initiator of the reactive hydrogen compound at the sites of the reactive hydrogen atoms and wherein chains of oxyethylene groups are then attached to the ends of the oxypropylene chains. Alternatively, the chains of oxyethylene groups having a defined molecular weight can be attached to the initiator of the reactive hydrogen compound at the sites of the reactive hydrogen atoms and then chains of oxypropylene groups can be attached to the ends of the oxyethylene chains.

The compounds for the compositions are prepared by condensing either ethylene oxide or propylene oxide with the reactive hydrogen on the initiator compound. After the first block of monomer units have been added to the initiator molecule, a second block of either propylene oxide or ethylene oxide is condensed with the reactive hydrogen on the end of the first block. It is to be understood that butylene oxide can be substituted, either all or part, for propylene oxide in the biologically active copolymers of the present invention.

It is to be noted that it is not necessary to use pure propylene oxide in producing the oxypropylene chains of the biologically active copolymers. Small amounts, for example, up to 5 weight percent, of ethylene oxide can be included in the propylene oxide employed to prepare the hydrophobic reactive hydrogen compound-propylene oxide condensate. Likewise, the ethylene oxide condensed with the hydrophobic propylene oxide-reactive hydrogen compound condensate can also contain small amounts, such as up to about 5 weight percent, of propylene oxide.

It is further to be noted that when molecular weight is stated in this specification and claims, unless otherwise noted, there is meant the average theoretical hydrophobe molecular weight which equals the total of the grams of the propylene oxide employed per mole of reactive hydrogen compound. It is well recognized in the field of alkylene oxide chemistry that the polyoxyalkylene compositions one obtains by condensing an alkylene oxide with a reactive hydrogen compound are actually mixtures of compounds rather than a single molecular compound. The mixture contains closely related homologues wherein the statistical average number of oxyalkylene groups equals the number of moles of the alkylene oxide employed and the individual members in the mixture contain varying numbers of oxyalkylene groups. Thus, the compositions of this invention are "mixtures" of compounds which are defined by molecular weight of the polyoxypropylene chains and weight percent of oxyethylene groups.

The biologically-active copolymers comprise a surface active compound with four hydrophobic segments and a small proportion of hydrophile. Typical examples have eight segments or octablock structure with a core of either a hydrophobic or hydrophilic central structure and a hydrophilic or hydrophobic outer structure as shown in the following schematic structures.

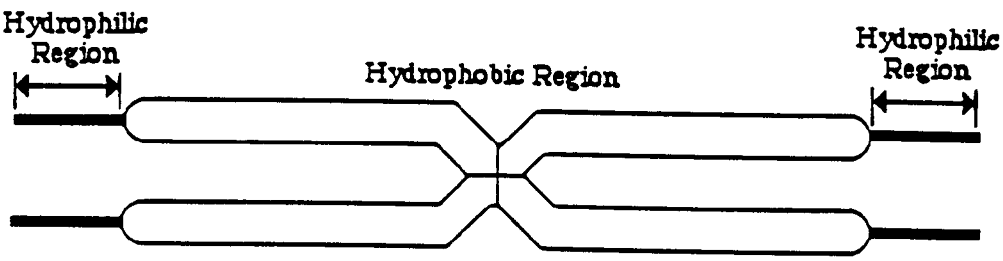


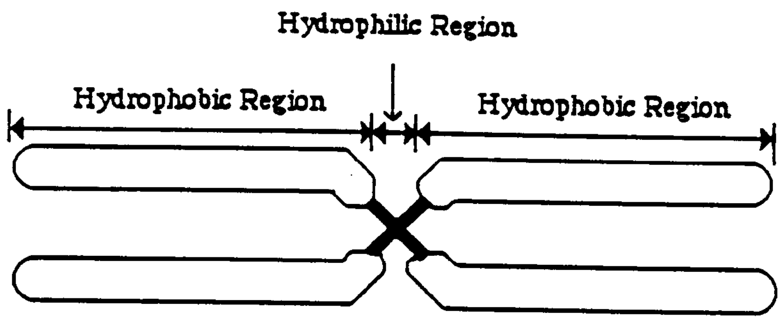


The entire molecule is poorly soluble in water and is either a nonionic or weakly cationic surface active agent. The steric configuration and physiochemical properties of the molecule, rather than the chemical nature of the constituent parts, are thought to be responsible for the biologic effects of the copolymer.

The biologically active compounds comprise blocks of polyoxypropylene and polyoxyethylene built on an alkylenediamine initiator. The blocks of polyoxypropylene (POP) and polyoxyethylene (POE) have the following structures:

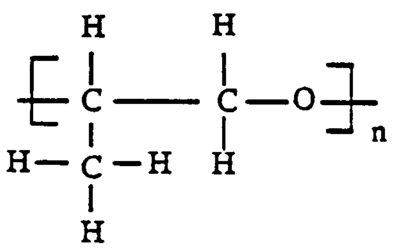

**Polyoxypropylene (POP)**

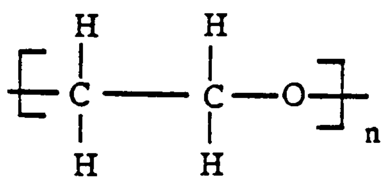

**Polyoxyethylene (POE)**

The polymer blocks are formed by condensation of ethylene oxide and propylene oxide onto a tetrafunctional ethylene diamine initiator at elevated temperature and pressure in the presence of a basic catalyst. There is some statistical variation in the number of monomer units which combine to form a polymer chain in each copolymer. The molecular weights given are approximations of the average weight of copolymer molecule in each preparation. A further description of the preparation of these block copolymers is found in U S -A- 2,674,619 and U S -A- 2,979,528. (Also see "A Review of Block Polymer Surfactants", Schmolka, I.R., *J. Am. Oil Chemists' Soc.*, 54:110-116(1977) and *Block and Graft Copolymerization*, Volume 2 edited by R.J. Ceresa, John Wiley & Sons, New York, 1976)

In one embodiment of the biologically active copolymers of the present invention, the block copolymer comprises a polymer of hydrophobic polyoxypropylene (POP) built on an ethylenediamine initiator. Polymers of hydrophilic polyoxyethylene (POE) are then built on the block of hydrophobic polyoxypropylene (POP). This results in an octablock copolymer with the following general formula:

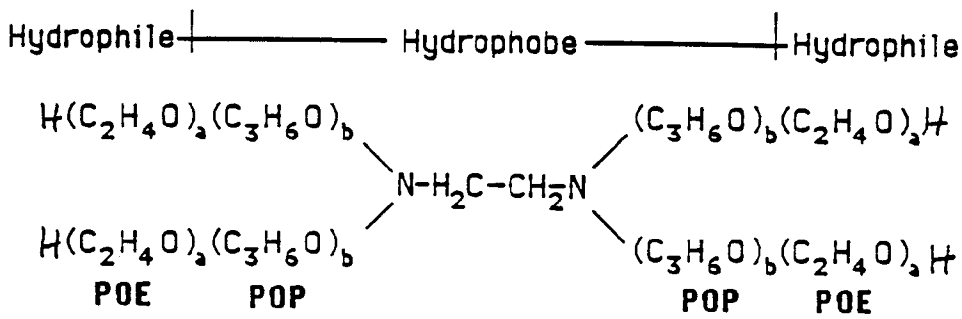

wherein:

the mean aggregate molecular weight of the hydrophobe portion of the octablock copolymer consisting of polyoxypropylene $(C_3H_6O)_b$ (POP) is between 5000 and 7000 daltons;

a is a number such that the hydrophile portion represented by polyoxyethylene $(C_2H_4O)_a$ (POE) constitutes between 10% to 40% of the total molecular weight of the compound;

b is a number such that the polyoxypropylene $(C_3H_6O)_b$ (POP) portion of the total molecular weight of the octablock copolymer constitutes between 60% and 90% of the compound; and

In another embodiment of the present invention, the block copolymer comprises a polymer of hydrophilic polyoxyethylene (POE) built on an ethylene diamine initiator. Polymers of hydrophobic polyoxypropylene (POP) are then built on the block of hydrophilic polyethylene (POE). This results in an octablock copolymer with the following general formula:

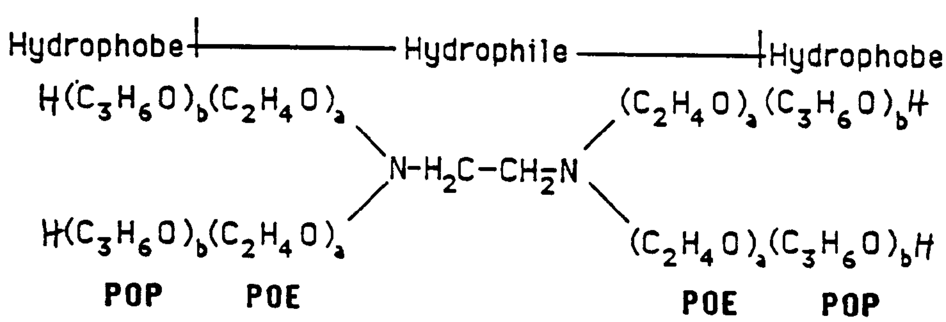

wherein:

The molecular weight of the hydrophobe portion of the octablock copolymer consisting of polyoxypropylene $(C_3H_6O)_b$ (POP) is between 5000 and 7000 daltons;

a is a number such that the hydrophile portion represented by polyoxyethylene $(C_2H_4O)_a$ (POE) constitutes between 10% and 40% of the total molecular weight of the compound;

b is a number such that the polyoxypropylene $(C_3H_6O)_b$ (POP) portion of the octablock copolymer constitutes between 60% and 90% of the compound; and

This type of polymer is called reverse copolymer because its structure is the reverse of octablock copolymers that have polyoxypropylene (POP) in the center flanked by blocks of polyoxyethylene (POE).

The octablock copolymers comprising the biologically active copolymers include the block copolymers Tetronic® and reverse Tetronic® manufactured by the BASF Corporation ((BASF Corporation, Parsippany, NJ).

A preferred biologically active copolymers is the octablock copolymer T130R2 (BASF Corporation, Parsippany, NJ) which corresponds to the following formula:

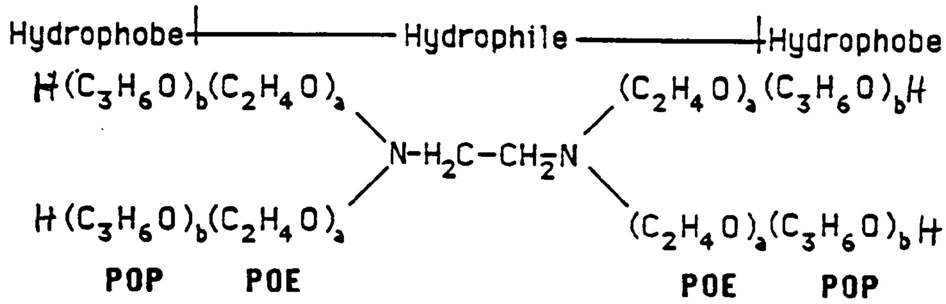

wherein:

The mean molecular weight of the hydrophobe portion of the octablock copolymer represented by polyoxypropylene $(C_3H_6O)_b$ (POP) is 5750 daltons;

a is a number such that the hydrophile portion represented by polyoxyethylene $(C_2H_4O)_a$ (POE) constitutes 20% of the compound by weight; and

b is a number such that the polyoxypropylene $(C_3H_6O)_b$ (POP) portion of the octablock copolymer constitutes 80% of the compound by weight.

Another preferred embodiment of the biologically active copolymers is the compound designated T1501 (BASF Corporation, Parsippany, NJ) which corresponds to the following formula:

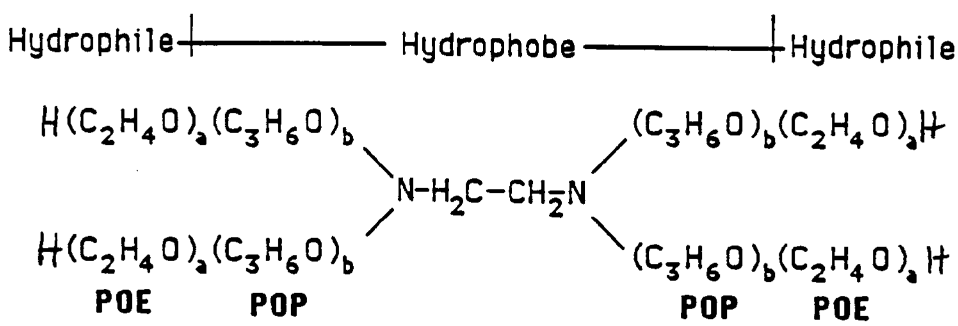

wherein:

the mean molecular weight of the hydrophobe portion of the octablock copolymer represented by polyoxypropylene $(C_3H_6O)_b$ (POP) is 6750 daltons;

a is a number such that the hydrophile portion represented by polyoxyethylene $(C_2H_4O)_a$ (POE) constitutes 10% of the compound by weight; and

b is a number such that the polyoxypropylene $(C_3H_6O)_b$ (POP) portion of the octablock copolymer constitutes 90% of the compound by weight.

The most preferred embodiment of the biologically active copolymers is the octablock copolymer T150R1 (BASF Corporation, Parsippany, NJ) which corresponds to the following formula:

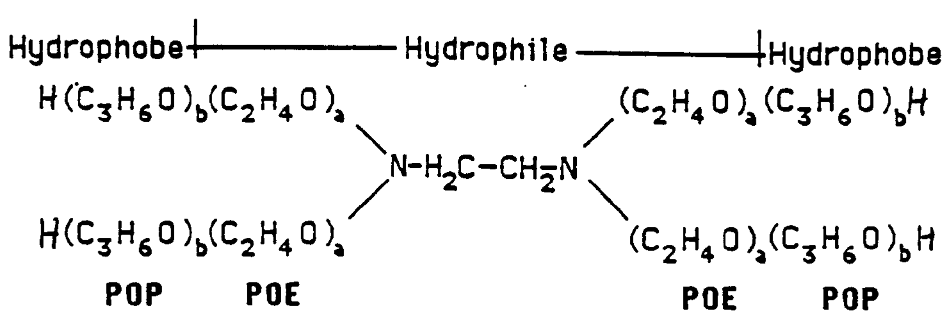

wherein:

The mean molecular weight of the hydrophobe portion of the octablock copolymer represented by

polyoxypropylene $(C_3H_6O)_b$ (POP) is 6750 daltons;

a is a number such that the hydrophile portion represented by polyoxyethylene $(C_2H_4O)_a$ (POE) constitutes 10% of the compound by weight; and

b is a number such that the polyoxypropylene $(C_3H_6O)_b$ (POP) portion of the octablock copolymer constitutes 90% of the compound by weight.

Although not wanting to be bound by the following theory, it is believed that the biologically active polymers of the present invention act by the following mechanism:

Investigators have demonstrated hormone-mediated interactions between the thymus and the central nervous system. The biologically active copolymers are capable of affecting this relationship. It is believed that the present invention acts by mimicking the conformation and physicochemical properties of natural hormones and neuropeptides. The biologic responses induced by the biologically active copolymers of the present invention include:

1. Gross morphologic changes in the uterus and adrenal glands;
2. Increased motor activity including excessive grooming;
3. Diuresis;
4. Noncytolytic release of histamine from mast cells by a temperature, calcium, energy dependent mechanism which is similar too from that of somatostatin and ACTH.

It is believed that there is a structural analogy between the biologically active copolymers and hormones, such as somatostatin, adrenocorticotropic hormone (ACTH), and ß-endorphin. These peptides have in common a sequence of basic amino acids adjacent to spans of hydrophobic amino acids. These features are thought to be essential for function. Interaction of the oligoamine residues with cell surface anions and stabilization by adjacent hydrophobic moieties initiates the molecular program resulting in effector functions of the peptide. The block polymers have the same structural features: a central basic ethylenediamine flanked by spans of hydrophobic polyoxypropylene (POP). By means of inhibition with monovalent amines, we have evidence that the central ethylenediamine groups function like the basic moieties of peptide hormones in stimulating release of histamine from mast cells.

*In vitro* studies provide direct evidence that the biologically active copolymers of the present invention act in a manner analogous to somatostatin and ACTH. Mast cells have been used as a model to study receptor mediated mechanisms through which these hormones induce their biological receptor mediated mechanisms. Minute quantities of the polymers cause histamine release from mast cells by a temperature-dependent process which requires energy and calcium and can be blocked by specific inhibitors in a manner very similar to that of somatostatin and ACTH.

Both agonistic and antagonistic effects of hormones may be elicited by the polymers. Biologic effects of the polymers vary with the structure of the polymer. The ability to modify the structure of these polymers to optimize particular biologic effects provides potential to design synthetic drugs with a precision and ease not possible in other systems.

Many chelating agents, such as ethylene-diaminetetraacetic acid (EDTA) consist of oligoamine sites flanked by hydrogen bonding groups. The addition of flanking hydrophobic moieties produces ionophores which are able to transport ions across lipid containing membranes. The compounds have this general structure and can act as ionophores transporting cations across artificial membranes. Consequently, the compounds represent a new chemical type of ionophore. Some neuropeptide hormones (*e.g.*, substance P) have ionophore activity.

The biologically-active copolymers are also effective in causing the cortex of the thymus to begin producing new T-lymphocytes thereby replenishing the immune system with these vital regulatory cells. The copolymers also induce proliferation of large numbers of post-thymic T-cells in the lymph nodes and other peripheral lymphoid tissues.

The biologically-active copolymers are generally poorly soluble in water. Although the compounds can be injected into an animal or human in aqueous media, it is preferable that the biologically-active copolymers used for the compositions of the present invention be injected as an oil-in-water or water-in-oil emulsion. A mineral oil or other oily substance such as Drakeol® 6VR or Drakeol® 5 (Penreco, Butler, PA) can be used as the oil phase of the emulsion. The aqueous phase can be physiologic phosphate buffered saline or other physiologic salt solution. The ratio of oil to water is preferably between 80:20 and 1:100.

Typically, an oil-in-water emulsion is prepared by mixing between approximately 0.5 to 50 grams of the biologically active copolymers with 5.0 ml of mineral oil in a Potter-Elvehjim homogenizer. Next, 95.0 ml of phosphate buffered saline (0.85 M sodium chloride. pH 7.3) containing 0.2% polyoxyethylene sorbitan monooleate (Tween® 80, Atlas Chemical Industries, Wilmington, DE) and 50 mg bovine serum albumin (BSA, Sigma Chemical Co., St. Louis, MO) is added. The mixture homogenized thoroughly to form a fine emulsion. The BSA and Tween® 80 are used to stabilize the emulsion. It is to be understood that the method of preparing the emulsion,

the proportions of oil and water and the type of oil used are not critical. An effective emulsion could be prepared by using a blender, by sonication or other means well known to those of ordinary skill in the art. It is to be further understood that other carriers, emulsifiers, aqueous solutions and adjuvants that are known to those of ordinary skill in the art can be used with the biologically active copolymers of the present invention.

Water-in-oil emulsions are prepared as follows. A stock oil-emulsifier mixture is prepared by blending mineral oil with 5% to 10% of a water-in-oil emulsifier. A mixture of 94.5% oil (Drakeol®, Penreco, Butler, PA), 4.5% Sorbitan monooleate (Span® 80, Atlas Chemical Industries, Wilmington, DE) and 0.5% polyoxyethylene sorbitan monooleate (Tween® 80, Atlas Chemical Industries, Wilmington, DE) is commonly used. A commercial blend, Freund's incomplete adjuvant, (Difco, Detroit, MI or Sigma Chemical, St. Louis, MO) is also suitable. 0.5 to 5.0 grams of the biologically active copolymers is added to either 60 ml of the oil-emulsifier mixture or to 40 ml of a physiologic saline solution similar to that used in the oil-in-water emulsion described above. The oil-emulsifier mixture is placed in a blender. The physiologic salt solution is added in three aliquots with vigorous homogenization to insure that a fine water-in-oil emulsion is prepared. Again, it is to be understood that the method of preparing the emulsion is not critical. Numerous variations of the composition of the aqueous and oil phases, their proportions and means of emulsification will be apparent to those skilled in the art and could be used with biologically active copolymers in practicting the invention.

The biologically active copolymers are effective with only one injection of compound being administered to an animal. However, in certain cases, subsequent injections may be necessary to achieve maximum stimulation of the immune system or other desired effect. The mode of injection of be subcutaneous, intramuscular or intravenous. A preferred mode of injection is subcutaneous. Intravenous injection is hazardous because of the toxic effects of embolic emulsions.

The optimum amount of the composition comprising biologically active copolymers in an injection varies with the size of the animal being treated. With animals such as rats or mice, the optimum amount of biologically active copolymers is between 0.5 and 5 mg per animal. With larger animals a larger quantity of biologically active copolymers is required in the injection for optimum results. With humans, cattle or swine, the dose varies with the age, size and condition of the individual but approximates 5 to 500 mg in most cases.

The following specific examples will illustrate the invention as it applies in particular to stimulating the immune system in mice and rats.

**Brief Description of the Figures**

FIG. 1 shows histamine release from mast cells that have been exposed to several copolymers,
FIG. 2 shows influx of sodium ions into red blood cells that have been exposed to several copolymers,
FIG. 3 shows the relationship between footpad swelling and sodium flux and several of the copolymers,
FIG. 4 shows the effect of T150R1 on mouse thymus *in vivo*.
FIG. 5 shows the effect of the copolymer on motor activity in rats.

**Examples**

**Mast Cell Degranulation**

The mast cell degranulation activity of several of the copolymers of the present invention are shown in this Example. Block copolymers were obtained from BASF Corporation, Parsipanny, NJ, and are shown in the following Table A.

**Table A**

| Copolymer | Mol. Wt.[a] | Mean[a,b] Structure |
|---|---|---|
| T130R1 | 6,800 | N-4-26 |
| T130R2 | 7,740 | N-10-26 |
| T150R1 | 8,000 | N-5-32 |
| T150R4 | 11,810 | N-27-32 |
| T150R8 | 20,400 | N-92-32 |

a Data from Manufacturer
b Amine nitrogen followed by mean number of ethoxy units (underlined) followed by mean number of propoxy units per linear chain

The copolymers in Table A range in physical form from viscous liquids to flakes. Measurement of the liquid copolymers was made using a positive displacement pipet based on a density approximately equal to that of water (range 1.01 - 1.03). Since the solubility of these materials in aqueous medium decreases with increasing temperature, all dilutions were made in cold (4°C) buffer.

Mouse peritoneal mast cells were obtained by lavage from female C3H mice (Charles River, Wilmington, MA). For each experiment one or two animals were sacrificed using dry ice vapors and the peritoneal cavity injected with 10 ml ice cold Dulbecco's phosphate-buffered saline (hereinafter PBS) (Gibco, Grand Island, NY) containing 10 U/ml heparin. The peritoneum was massaged for 30 seconds and the fluid withdrawn. Cells were washed twice in PBS and counted using Alcian blue. The average yield was 4-6 x $10^6$ cells/animal with mast cells comprising 3-5% of the total cells. Cells were resuspended in Tyrode's solution (see Pearce, F.L. and White, J.R. (1984) *Agents Actions* 14, 392) containing 1 mg/ml bovine serum albumin (Miles Laboratories, Naperville, IL). Tyrode's solution was made using distilled, deionized water with 137 mM NaCl, LiCl, or KCl as the major cation species with 1 mM $CaCl_2$ added as indicated.

Cells were prewarmed to 37°C for 5 minutes and then added in 200 µl aliquots (2-5 x $10^3$ mast cells) to polypropylene tubes containing an equal volume of buffer with or without copolymer. Total histamine content of the cells was obtained by lysing the cells in 3% perchloric acid. Cells were incubated for 30 minutes at 37°C and then centrifuged for 5 minutes at 1500 x g at 4°C. Supernatants were analyzed for histamine content using the automated fluorometric method (See Siraganian, R.P. (1974) *Anal. Biochem.* 57, 383). All samples were performed in duplicate and the results are presented as means of sample pairs. Net percent histamine release was calculated by the following formula:

All of the copolymers shown in Table A were tested for their ability to cause non-cytolytic histamine release. The five copolymers were incubated at a concentration of 100 µg/ml with the mast cells. At the end of 30 minutes incubation with the copolymer, the per cent net histamine release was measured. These results are summarized in FIG. 1. As shown, the rates obtained for these copolymers revealed a spectrum of activity with the T130R2 copolymer showing the greatest histamine release and the T150R8 showing the least activity.

**Ionophore Activity**

The ionophore activity of the biologically active copolymers was determined by measuring the influx of ions into human red blood cells.

Human blood anticoagulated with lithium heparin was washed 3 times in 0.9% NaCl by centrifugation for 10 minutes at room temperature at 150 x g and diluted to 10% hematocrit in saline. For $Na^+$ flux measurements the saline contained 5µCi $^{22}Na^+$/ml (Amersham Corp., Arlington Heights, IL). For determination of $Ca^{++}$ flux, cells

were diluted in saline containing 4 mM CaCl2 with $^{45}Ca^{++}$ tracer (ICN Biomedicals, Inc., Irvine, CA) at 10 μCi/ml. Cells were warmed to 37°C in a water bath and then added to equal volumes of pre-warmed buffer containing copolymers and mixed by vortexing. For $Na^+$ or $Ca^{++}$ flux measurements, 50μl duplicate aliquots of each whole suspension were removed during incubation to measure total activity. At selected time points, 200μl duplicate aliquots were removed and added to 1.5 ml minicentrifuge tubes (American Scientific Products, McGaw Park, IL) containing 200 μl SF1154 silicone oil (General Electric Co., Waterford, NY).

For $Na^+$ and $Ca^{++}$ measurements, the supernatants above the oil were aspirated and the portion of the tube above the oil was washed twice by carefully adding and then aspirating distilled water. After removal of most of the oil the cells were resuspended in 250 μl distilled water. Residual $^{22}Na^+$ activity was measured using a gamma counter and remaining $^{45}Ca^{++}$ activity was measured in a beta counter after mixing 100 μl of the lysate with 3 ml Opti-Fluor LSC cocktail (United Technologies Packard). Determination of potassium efflux was performed as above except that the cells were mixed with copolymer in normal saline at a 10% final hematocrit. After centrifugation, supernatants were removed and assayed for potassium content by flame emission spectroscopy using an IL 443 Flame Photometer (Instrumentation Laboratory, Inc., Lexington, MA).

Residual counts in the pellets or the amount of $K^+$ in the supernatants were normalized for pellet hemoglobin content which was determined using Drabkin's solution. There was no difference between hemoglobin content of pellets incubated with any of the copolymers used or with buffer alone, indicating that copolymers are not cytolytic. All experiments were performed at least in duplicate.

Five copolymers were tested for their ability to cause influx of $Na^+$ ions over a period of 30 minutes. The results of these tests are summarized in FIG. 2.

Comparison of the rates obtained for these copolymers revealed a spectrum of activity which correlated with their ability to trigger in vitro histamine release from murine mast cells and *in vivo* inflammation as determined by peak footpad swelling following subplantar injection in mice. These results are summarized in FIG. 3.

The larger the sodium flux observed with each copolymer, the more histamine release and inflammation the copolymer induced.

**Histamine Release vs. Footpad Swelling**

Three octablock and seven reverse octablock copolymers were obtained from the BASF, Parsipanny, NJ. Resident peritoneal leukocytes were obtained by lavage as described in Example 7.2.

Six of seven reverse octablock copolymers were found to release significant amounts of histamine from mouse peritoneal mast cells. These results are summarized in Table B.

**Table B**
**Histamine Release Compared to Footpad Swelling Induced by Copolymers**

| Copolymer | Ave.[a] M.W. | Chain[b] Structure | %Histamine Release[c] 10μg/ml | 100μg/ml | Peak Footpad[d] Swelling (mm) |
|---|---|---|---|---|---|
| T130R2 | 7,740 | N-10-26 | 58.0±8.6 | 66.3±6.5 | 2.92±0.41 |
| T130R1 | 6,800 | N-4-26 | 16.3±4.0 | 29.0±7.0 | 1.65±0.3 |
| T110R1 | 5,220 | N-3-21 | 16.7±2.6 | 15.7±2.6 | 1.3±0.33 |
| T90R1 | 4,580 | N-3-18 | 15.7±2.6 | 13.3±2.5 | 1.18±0.24 |
| T150R1 | 8,000 | N-5-32 | 9.0±0.8 | 11.7±3.1 | 1.17±0.08 |
| T150R4 | 11,810 | N-27-32 | 0.3±0.5 | 16.3±7.9 | 1.04±0.5 |
| T150R8 | 20,400 | N-92-32 | 0.7±0.5 | 0.0±0.0 | 0.79±0.01 |

a Average Molecular Weight (data from manufacturer)

b Reverse octablocks are 4-chain structures arranged around a core of ethylenediamine moiety. Chain structure is here represented by the amine nitrogen (N) followed by the average number of POE blocks (underlined) and the number of POP blocks in one chain.

c Mean histamine content of supernatants from cells incubated for 30 minutes at 37°C with copolymers at the indicated concentrations. Mean ± standard deviation for three experiments. Values are corrected for spontaneous release which was always less than 10%.

d Rank order of peak footpad swelling produced by subplantar injection of 50μl of an oil in water emulsion containing 1.25 mg copolymer (mean ± SE on groups of 10 mice.)

The amount of histamine released by the various copolymers was related to the level of inflammation at the site of injection, i.e., the more inflammatory the copolymer *in vivo*, the more histamine release *in vitro*.

**Thymus Stimulation Activity**

Six week old female ICR outbred mice were injected in the rear footpads with an oil-in-water emulsion containing copolymer 1.25 mg T105R1, 2.5 mg mineral oil and 25μg bovine serum albumin in 0.05 ml of 0.01 M phosphate buffered saline containing 0.1% Tween® 80. Identical injections were made into both rear footpads. Controls were animals that were injected with emulsions without the T150R1. Animals were sacrificed at 4 days, 1, 2, 3 and 6 weeks. Their thymuses were carefully dissected free of connective tissue and were weighed.

Microscopic examination revealed that the changes in the thymus were limited almost exclusively to the cortical lymphocytes. Their numbers were markedly reduced three days after injection, but approached normal at one week. After the transient decrease in the number of lymphocytes, the size of the thymus glands of treated animals increased markedly over those of controls. The medullary areas demonstrated little change, but the cortical areas were much larger than normal. The cortical areas of the thymus were composed of immature and mature small lymphoid cells. The reduction in size of the thymus in controls was the expected result of normal involution with aging. Thus, the injection of T150R1 prevented involution during the time studied. In several experiments carried out with identical protocol, the thymuses of treated animals was consistently nearly twice as large as those of the controls. Some treated mice were followed for over eighteen months and appear

healthy.

Table C shows that the inhibition of involution of the thymus is long term. The weights of the body, thymus and spleens were after 18 months.

### Table C

| | Control (n=3) | injected at 4 weeks (n=3) | injected at 6 weeks (n=3) |
|---|---|---|---|
| body | 40.7±7g | 47.0±4.2g | 53.3±1.6g |
| thymus | 34.7±5.0 mg | 67.6±18.7 mg | 66.2±26 mg |
| spleen | 126.7±47.3 mg | 180.7±61.0 mg | 122.7±19 mg |

Histological examination of the thymuses showed little thymus tissue in control mice. Thymuses of injected mice had "young looking" thymus tissue.

**Thymus Stimulation, Copolymer in Saline**

Mice were injected via the tail vein with the following preparations:

1. Saline at 4°C
2. 2.5 mg of copolymer T150R1 in saline at 4°C
3. 10μ beads coated with T150R1 copolymer.

Each experimental group contained four mice. After 6 days, the mice were sacrificed and the thymuses were carefully dissected and weighed. The results of this experiment are summarized in FIG. 4.

This Example shows that the copolymer dissolved in cold saline and injected directly into the blood of an animal causes a marked increase in the weight of the thymus.

**Thymus Stimulation, Oil and Water Emulsion**

Mice were injected in the footpad with related copolymers and other forms of emulsion. Copolymers T130R2 and T1501 had weaker effects in stimulating the increased size of the thymus. The results of an experiment with water in oil emulsions is shown in Table D.

## Table D

| Copolymer | WATER-IN-OIL EMULSIONS Weight of the thymus in mg 2 weeks | 6 weeks |
|---|---|---|
| T150R1 | 65 ± 7 | 110 ± 7 |
| T1501 | 84 ± 15 | 77 ± 4 |
| Vehicle Control | 101 ± 18 | 50 ± 7 |

### Thymus Stimulation, General Observations

Mice were treated as dealt with in the paragraphe Thymus Stimulation, Oil and Water Emulsion and carefully observed for a period of several months. The following observations were made. The treated mice were larger than control mice. The treated mice appeared to exhibit increased motor activity including more general activity in the cage, excessive grooming and the treated mice ate more than the control mice.

The uterus of the treated femalee mice was larger and appeared to have increased vascularity. There was an increased prominence of Peyer's patches (gut associated lymphoid tissue) in the treated animals.

### Stimulation of Motor Activity

The effect the biologically-active copolymer has on the motor activity of rats was measured. The animals used in this Example were adult male Sprague-Dawley rats.

The rats were individually housed in suspended cages. All rats were housed for three weeks in the same room prior to initiation of the study.

Baseline measurements of horizontal locomotor activity were made for one week prior to administration of the copolymer. Horizontal activity was measured using an activity box (Omnitech, Inc., Columbus, Ohio) interfaced with a Vic 20 microcomputer. The activity box has two rows of 8 photocells positioned at 90° angles. When the light path to a photocell is broken, a voltage drop occurs and is recorded by the computer. Activity determinations were made on all nine rats at three day intervals.

The test and control solutions were prepared as follows: The aqueous phase was prepared by mixing 100 ml of PBS and 0.2 ml of Tween®-80. This mixture was stirred approximately 5 minutes. 200μl of oil (Drakeol®, Penreco, Butler, Pa) was added to 100μl of T150R1. This mixture of biologically active copolymer and oil was homogenized for two minutes in a Potter-Elvinjim homogenizer. Next, 1 ml of PBS with 2%Tween®-80 was added and homogenized for another 2 minutes. Finally, an additional 1 ml of PBS with 2%Tween®-80 was added and homogenized for 2 minutes. The control emulsion was prepared as above but without the addition of T150R1 copolymer.

All rats were injected with 100 μl of either the control or the test emulsion. The test emulsion contained a total of 5mg of T150R1 copolymer. All injections were given subcutaneouslv on the dorsum of the neck. The effect of the compound on the activity of rats is shown in FIG. 5.

As shown in FIG. 5 , rats that were injected with the biologically active copolymer of the present invention demonstrated markedly increased activity over the control.

### Claims

1. Use of biologically active octablock copolymers having the following formula

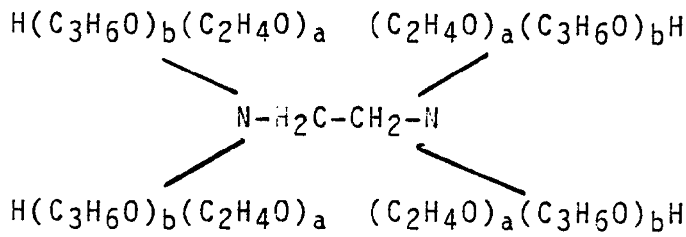

and

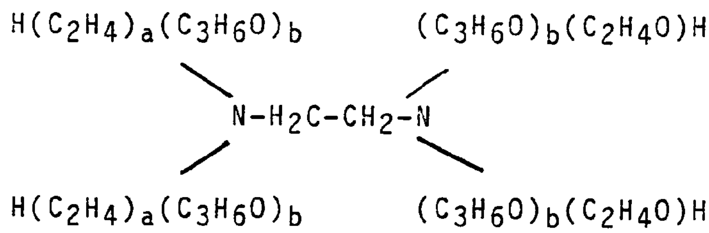

wherein:

the mean aggregrate molecular weight of the portion of the octablock copolymer represented by polyoxypropylene is between 5000 and 7000 daltons;

a is a number such that the portion represented by polyoxyethylene constitutes between 10 % to 40 % of the compound by weight; and

b is a number such that the polyoxypropylene portion of the total molecular weight of the octablock copolymer constitutes between 60 % and 90 % of the compound by weight for the preparation of a pharmaceutical composition for stimulating the immune system of an animal or human.

2. Use of claim 1, wherein said octablock copolymer has the following structure:

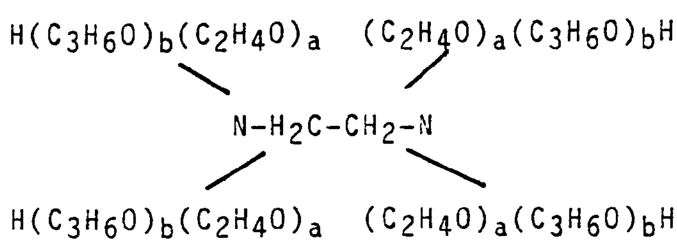

wherein

the mean aggregrate molecular weight of the portion of the octablock copolymer represented by polyoxypropylene is 6750 daltons;

a is a number such that the portion of the total molecular weight represented by polyoxyethylene constitutes 10 % of the compound by weight; and

b is a number such that the polyoxypropylene portion of the octablock copolymer constitutes 90 % of the compound by weight.

## Patentansprüche

1. Verwendung von biologisch aktiven Octablockcopolymeren der folgenden Formel

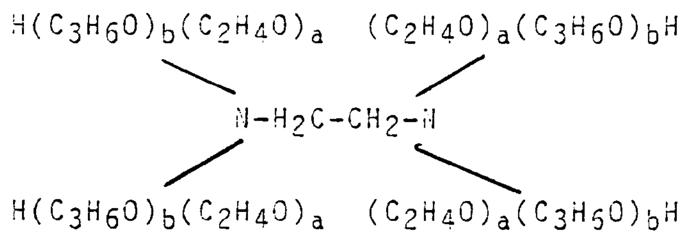

und

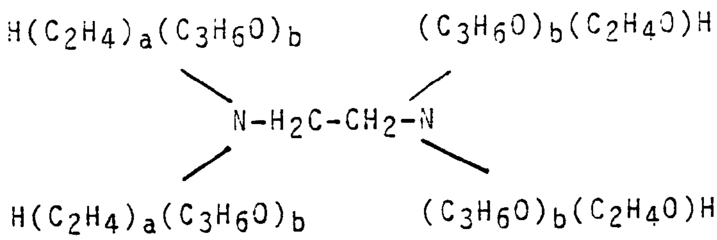

in denen das durchschittliche aggregierte Molekulargewicht des Teils des Octablockcopolymeren, das durch Polyoxypropylen gebildet wird, zwischen 5000 und 7000 Dalton beträgt,

a eine Zahl ist, so daß der Polyoxyethylenanteil zwischen 10 und 40 Gew.-% der Verbindung ausmacht, und

b eine Zahl ist, so daß der Polyoxypropylenanteil des gesamten Molekulargewichts des Octablockcopolymeren zwischen 60 und 90 Gew.-% der Verbindung ausmacht, zur Herstellung einer pharmazeutischen Zusammensetzung für die Anregung des Immunsystems eines Tieres oder Menschen.

2. Verwendung nach Anspruch 1, **dadurch gekennzeichnet**, daß das Octablockcopolymere die folgende Struktur hat

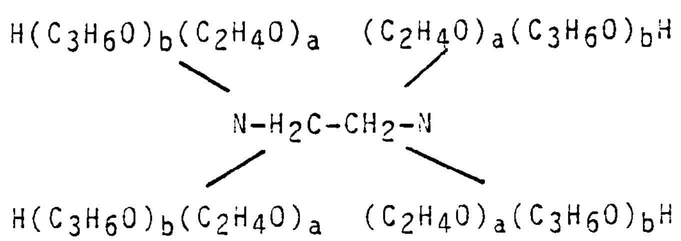

in der das durchschittliche aggregierte Molekulargewicht des Teils des Octablockcopolymeren, das durch Polyoxypropylen gebildet wird, 6750 Dalton beträgt,

a eine Zahl ist, so daß der Polyoxyethylenanteil des gesamten Molekulargewichts 10 Gew.-% der Verbindung ausmacht, und

b eine Zahl ist, so daß der Polyoxypropylenanteil des Octablockcopolymeren 90 Gew.-% der Verbindung ausmacht.

**Revendications**

1. Utilisation de copolymères octablocs biologiquement actifs ayant la formule suivante :

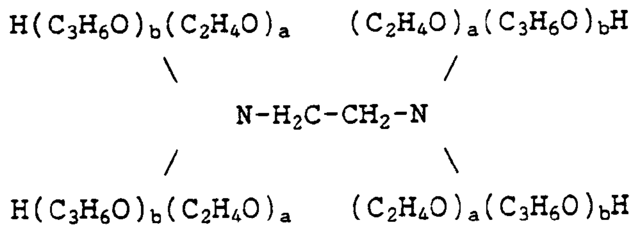

et

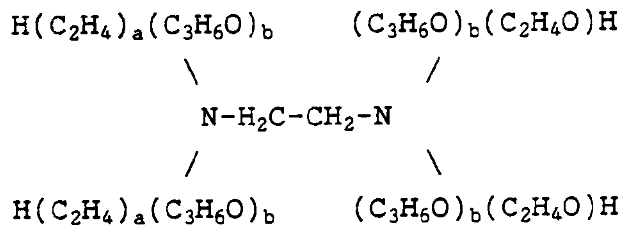

dans laquelle le poids moléculaire moyen d'agrégat de la portion du copolymère octabloc représentée par le polyoxypropylène est compris entre 5000 et 7000 daltons :

a) est un nombre tel que la portion représentée par le polyoxyéthylène constitue entre 10 et 40 % du composé en poids et,

b) est un nombre tel que la portion de polyoxypropylène du poids moléculaire total du copolymère octabloc constitue entre 60 et 90 % du composé en poids pour la préparation d'une composition pharmaceutique destinée à stimuler le système immun d'un animal ou de l'homme.

2. Utilisation selon la revendication 1, dans laquelle ledit copolymère octabloc a la structure suivante :

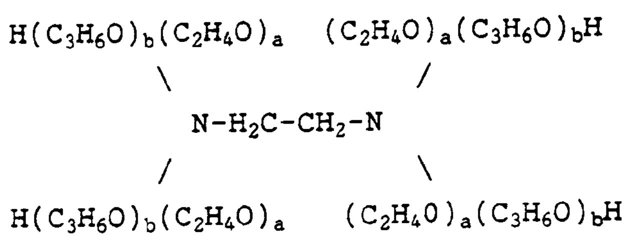

dans laquelle :

le poids moléculaire moyen d'agrégat de la portion du copolymère octabloc représentée par le polyoxyropylène est de 6750 daltons ;

a) est un nombre tel que la portion de poids moléculaire total représenté par le polyoxyéthylène constitue 10 % du composé en poids et,

b) est un nombre tel que la portion polyoxypropylène du copolymère octabloc constitue 90 % en poids du composé.

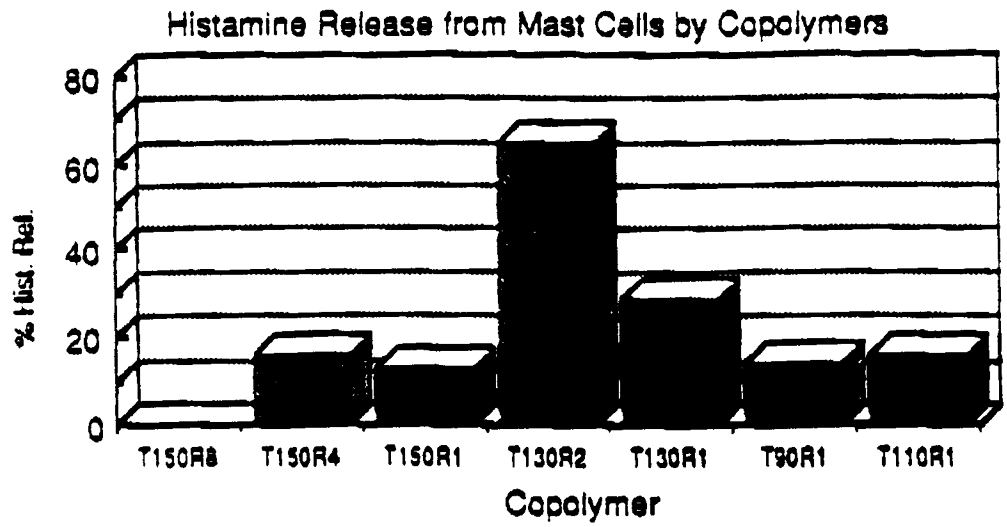
Histamine Release from Mast Cells by Copolymers
% Hist. Rel.
80
60
40
20
0
T150R8
T150R4
T150R1
T130R2
T130R1
T90R1
T110R1
Copolymer

Fig. 1

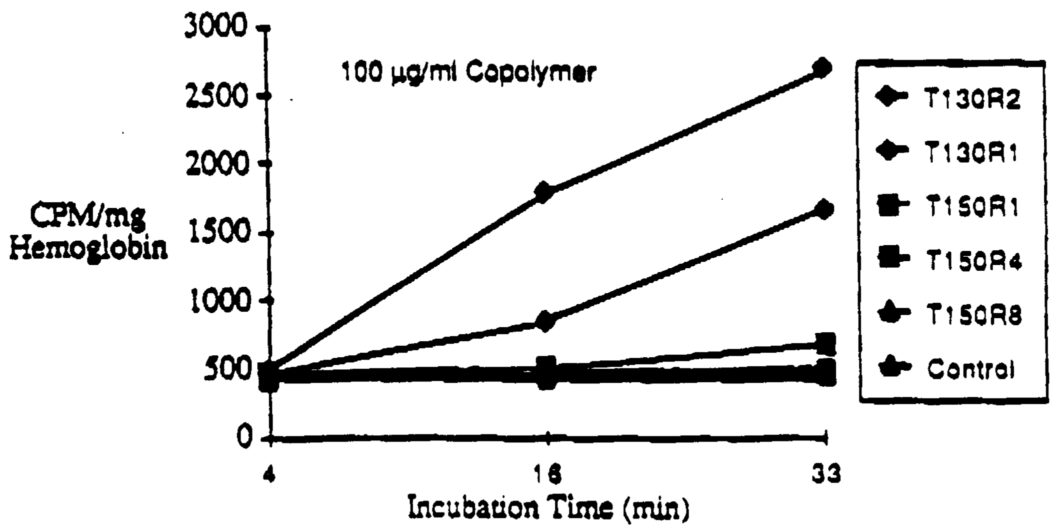
3000
2500
2000
1500
1000
500
0
CPM/mg Hemoglobin
100 µg/ml Copolymer
4
16
33
Incubation Time (min)
T130R2
T130R1
T150R1
T150R4
T150R8
Control

FIG. 2

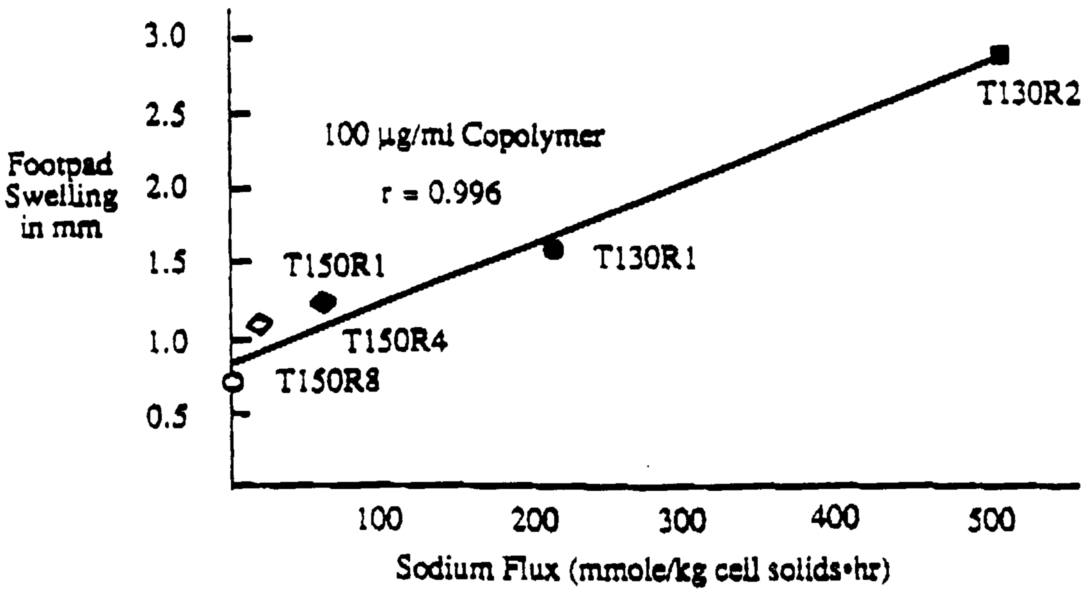
3.0
2.5
2.0
1.5
1.0
0.5
Footpad Swelling in mm
100 µg/ml Copolymer
r = 0.996
T130R2
T130R1
T150R1
T150R4
T150R8
100
200
300
400
500
Sodium Flux (mmole/kg cell solids•hr)

FIG. 3

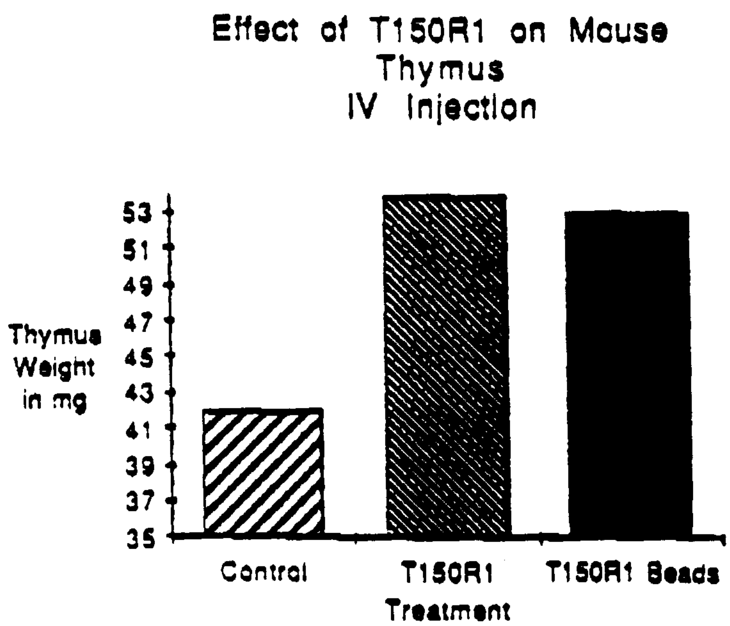
Effect of T150R1 on Mouse
Thymus
IV Injection
Thymus Weight in mg
53
51
49
47
45
43
41
39
37
35
Control
T150R1
T150R1 Beads
Treatment

FIG. 4

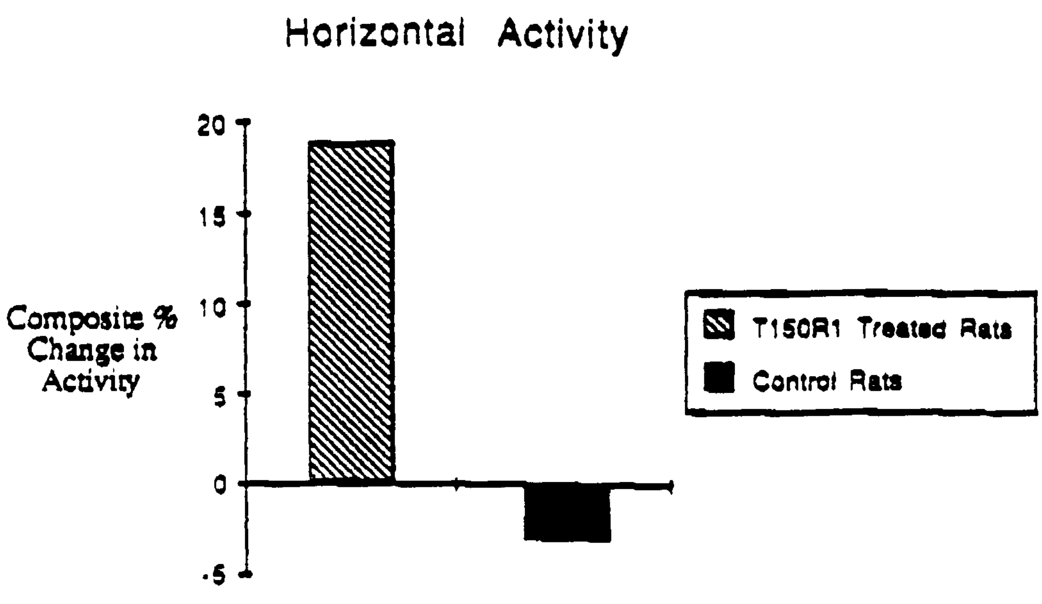
Horizontal Activity
20
15
10
5
0
-5
Composite % Change in Activity
T150R1 Treated Rats
Control Rats

Fig. 5